(19) Europäisches Patentamt
European Patent Office
Office européen des brevets

(11) EP 3 597 130 A1

(12) EUROPÄISCHE PATENTANMELDUNG

(43) Veröffentlichungstag:
22.01.2020  Patentblatt 2020/04

(51) Int Cl.:
A61B 17/88 (2006.01)          A61F 2/46 (2006.01)
A61B 17/70 (2006.01)          A61C 9/00 (2006.01)

(21) Anmeldenummer: 19196674.6

(22) Anmeldetag: 17.12.2014

(84) Benannte Vertragsstaaten:
AL AT BE BG CH CY CZ DE DK EE ES FI FR GB
GR HR HU IE IS IT LI LT LU LV MC MK MT NL NO
PL PT RO RS SE SI SK SM TR

(62) Dokumentnummer(n) der früheren Anmeldung(en)
nach Art. 76 EPÜ:
14824438.7 / 3 232 965

(71) Anmelder: Sulzer Mixpac AG
9469 Haag (Rheintal) (CH)

(72) Erfinder:
• GRETER, Andy
6648 Minusio (CH)

• NIEBER, Benjamin
6274 Eschenbach (CH)
• PROCTER, Philip
01220 Divonne les Bains (FR)

(74) Vertreter: Piticco, Lorena
Isler & Pedrazzini AG
Giesshübelstrasse 45
Postfach 1772
8027 Zürich (CH)

Bemerkungen:
Diese Anmeldung ist am 11.09.2019 als
Teilanmeldung zu der unter INID-Code 62 erwähnten
Anmeldung eingereicht worden.

(54) AUSTRAGVORRICHTUNG FÜR KNOCHENERSATZMATERIALIEN

(57) Eine Austragvorrichtung (1) zum Austragen einer Masse, z.B. eines Knochenersatzmaterials, weist ein Gehäuse (200) und einen darin verdrehbar angeordneten Behältereinsatz (300) auf. In einer Gehäusewand (204) ist eine laterale Aussparung (205) ausgebildet, und in einer Behälterwand (304) ist eine laterale Behälteröffnung (303) ausgebildet. Der Behältereinsatz ist relativ zum Gehäuse zwischen einer Verschliessstellung und einer Freigabestellung verdrehbar. Ein Kolben dient dazu, ddie Masse aus dem Behältereinsatz auszutragen. Um zu verhindern, dass die Masse beim Austragen komprimiert wird, erstreckt sich die laterale Behälteröffnung durchgehend bis zum distalen Behälterende (302), ohne dass sich entlang der Längsachse in distaler Richtung ein Bereich der Behälterwand an die laterale Behälteröffnung anschliesst. Um das Einfüllen der Masse zu erleichtern, nimmt die Wandstärke der Gehäusewand zur lateralen Aussparung (205) hin kontinuierlich ab. Um ein Blockieren des Kolbens zu verhindern, weitet sich der Querschnitt des Behältereinsatzes in distaler Richtung kontinuierlich auf.

FIG. 1

**Beschreibung**

TECHNISCHES GEBIET

Die vorliegende Erfindung betrifft eine Austragvorrichtung zum Austragen einer nur schlecht fliessfähigen Masse, insbesondere eines Knochenersatzmaterials (Bone-Graft-Materials), mit einem Gehäuse und einem darin angeordneten Behältereinsatz, wobei in einer Gehäusewand des Gehäuses eine laterale Aussparung ausgebildet ist, wobei in einer Behälterwand des Behältereinsatzes eine laterale Behälteröffnung ausgebildet ist, und wobei der Behältereinsatz relativ zum Gehäuse zwischen einer Verschliessstellung, in der die Gehäusewand die laterale Behälteröffnung verschliesst, und einer Freigabestellung, in der die laterale Aussparung die laterale Behälteröffnung freigibt, verdrehbar ist.

STAND DER TECHNIK

In verschiedenen medizinischen Bereichen wie der Zahnheilkunde, der Orthopädie oder der rekonstruktiven Chirurgie kommen Knochenersatzmaterialien (Bone-Graft-Materialien) zum Einsatz. Knochenersatzmaterialien können eingeteilt werden in sogenannte Autografts (Knochentransplantat, das aus patienteneigenem Knochenmaterial hergestellt ist), Allografts (Knochentransplantat, das aus patientenfremdem humanem Knochenmaterial hergestellt ist, z.B. aus einer Knochenbank stammend), Xenografts (aus biologischem Material nichthumanen Ursprungs hergestellt) und synthetische Knochenersatzmaterialien (z.B. auf der Basis von Hydroxyapatit, häufig unter Beimischung von Wachstumsfaktoren und körpereigenen Substanzen).

In manchen Anwendungen liegt das Knochenersatzmaterial in Form einer schlecht fliessfähigen Masse vor. Diese besteht meist aus einer partikelförmigen oder granularen festen Phase (z.B. gemahlene oder anderweitig zerkleinerte feste Knochenbestandteile und/oder pulverförmige synthetische Materialien wie Hydroxyapatit, Trikalziumphosphat, Kalziumsulfat oder Bioglas) und einer flüssigen Phase (z.B. körpereigenes Blut, Blutplasma, Knochenmarkaspirat usw.). Eine solche Masse erinnert häufig in ihrer Konsistenz an feuchten Sand. Zudem können grössere Partikel wie z.B. Knochensplitter in der Masse vorkommen. Bei Kompression neigt eine solche Masse zum Zusammenbacken und verliert ihre Fliessfähigkeit fast vollständig. Die Masse kann je nach Zusammensetzung und Flüssigkeitsgehalt eine eher pulverartige oder eher pastöse Konsistenz haben. Sie ist mit herkömmlichen Injektionsvorrichtungen nicht injizierbar.

Die Handhabung und die Verabreichung einer solchen Masse an ihren Bestimmungsort können mit einigen Schwierigkeiten verbunden sein. Traditionell wird die Masse vom Chirurgen von Hand oder mit einem Spatel an den Bestimmungsort verbracht. Je nach Lage des Bestimmungsorts kann dies aber schwierig bis unmöglich sein. Es besteht daher ein Bedürfnis, Massen der hier genannten Art mit Hilfe von spritzenartigen Austragvorrichtungen an ihren Bestimmungsort, der insbesondere im Körperinneren liegen kann, zu bringen. Bei der Masse kann es sich, wie schon dargelegt, um ein Knochenersatzmaterial handeln, es kann sich jedoch auch im eine andere Art von Material handeln, die in das Innere eines menschlichen oder tierischen Körpers eingebracht werden soll.

Im Stand der Technik wurden hierfür verschiedene Austragvorrichtungen vorgeschlagen. Ebenfalls wurden verschiedene Verfahren vorgeschlagen, um eine solche Austragvorrichtung mit einer Masse zu befüllen. Sowohl die Befüllung als auch die anschliessende Verabreichung können aber wegen der speziellen Konsistenz einer Masse der genannten Art schwierig sein.

Die US 8,034,034 B2 offenbart eine Spritze mit einer lateralen Öffnung. Injizierbares Material wie z.B. Knochenzement kann durch die Öffnung in die Spritze eingefüllt werden. Nach dem Befüllen wird die Öffnung durch einen Deckel verschlossen. Es werden mehrere Möglichkeiten zum Verschliessen der Öffnung offenbart; so kann sich der Deckel z.B. bei Betätigung des Spritzenkolbens über die Öffnung schieben, oder er wird bei Betätigung des Spritzenkolbens auf die Öffnung verschwenkt. Es ist auch offenbart, die Öffnung unabhängig von einer Betätigung des Spritzenkolbens zu verschliessen. Eine solche Spritze eignet sich jedoch nur schlecht zum Austragen von Knochenersatzmaterialien mit einer stark partikelhaltigen Konsistenz. In der Praxis kann der Gegendruck durch das Knochenersatzmaterial so gross werden, dass ein Austragen des Materials völlig verunmöglicht wird.

Die US 6,582,438 offenbart eine Austragvorrichtung, welche über zwei konzentrische Zylinder mit länglichen Öffnungen in ihren Seitenwänden und einen Kolben verfügt. Um ein Knochenersatzmaterial in die Zylinder einzufüllen, werden die Zylinder derart gegeneinander verdreht, dass ihre Öffnungen übereinander zu liegen kommen. Das Knochenersatzmaterial wird sodann lateral eingefüllt. Nach dem Befüllen wird der äussere Zylinder gedreht, so dass er mit seiner Seitenwand die Öffnung des darunter liegenden inneren Zylinders schliesst. Das Knochenersatzmaterial wird ausgetragen, indem der Kolben durch den inneren Zylinder durchgestossen wird. Auch bei dieser Austragvorrichtung zeigt sich allerdings in der Praxis, dass das Austragen von stark partikelhaltigen Knochenersatzmaterialien mit einem sehr hohen Gegendruck verbunden sein kann, bis hin zu einem völligen Blockieren des Kolbens. Die Ursache hierfür war bislang unbekannt.

Zudem können grössere Partikel im auszutragenden Material, wie beispielsweise Knochensplitter, schon beim Befüllen dazu führen, dass sich die laterale Öffnung nicht mehr richtig verschliessen lässt, und beim Austragen können solche Partikel zusätzlich zu einer Blockierung des Kolbens führen.

DARSTELLUNG DER ERFINDUNG

In einem ersten Aspekt ist es eine Aufgabe der vorliegenden Erfindung, eine Austragvorrichtung zum Austragen einer Masse, insbesondere eines Knochenersatzmaterials, anzugeben, bei welcher ein Verklemmen des Kolbens beim Austragen der Masse durch grössere Partikel wie Knochensplitter vermieden wird.
Diese Aufgabe wird durch eine Austragvorrichtung mit den Merkmalen des Anspruchs 1 gelöst.
Es wird also eine Austragvorrichtung zum Austragen einer Masse angegeben, welche aufweist:

ein Gehäuse mit einer umlaufenden Gehäusewand, einem offenen proximalen Gehäuseende und einem offenen distalen Gehäuseende, wobei eine laterale Aussparung in der Gehäusewand ausgebildet ist; und
einen im Gehäuse angeordneten Behältereinsatz mit einer umlaufenden Behälterwand, mit einem offenen proximalen Behälterende und einem distalen Behälterende, wobei in der Behälterwand eine laterale Behälteröffnung ausgebildet ist, und wobei der Behältereinsatz relativ zum Gehäuse zwischen einer Verschliessstellung, in der die Gehäusewand die laterale Behälteröffnung verschliesst, und einer Freigabestellung, in der die laterale Aussparung die laterale Behälteröffnung freigibt, um eine Längsachse verdrehbar ist.

Um zu vermeiden, dass ein Kolben beim Durchschieben durch den Behälter durch grössere Partikel wie Knochensplitter verklemmt wird, weist der Behältereinsatz einen freien Behälterquerschnitt auf, der entlang der Längsachse über einen axialen Bereich in distaler Richtung kontinuierlich und bevorzugt linear zunimmt.
Die kontinuierliche Zunahme des freien Behälterquerschnitts erfolgt dabei vorzugweise um einen Betrag, der sich wie folgt berechnet: Im Falle eines innenseitig rotationssymmetrischen Behälters nimmt der Innendurchmesser pro Längeneinheit um mindestens 0.5 Promille zu, d.h. pro Zentimeter Länge des Behälters nimmt der Innendurchmesser um mindestens 5 Mikrometer zu. Vorzugsweise beträgt die Zunahme mindestens 2 Promille. Vorzugsweise beträgt die Zunahme andererseits höchstens 30 Promille, d.h. der pro Zentimeter Länge des Behälters nimmt der Innendurchmesser um höchstens 0.3 Millimeter zu.
Im allgemeinen Falle eines innenseitig nicht notwendig rotationssymmetrischen Behälters nimmt entsprechend eine Grösse $\langle D \rangle$ pro Längeneinheit um mindestens 0.5 Promille, vorzugsweise mindestens 2 Promille, und um höchstens 30 Promille zu, wobei die Grösse $\langle D \rangle$ einen mittleren Durchmesser bezeichnet und wie folgt definiert ist:

$$\langle D \rangle = 2\sqrt{A/\pi},$$

wobei A die freie Querschnittsfläche des Behälters bezeichnet.
Der axiale Bereich, über den hinweg der freie Behälterquerschnitt zunimmt, erstreckt sich vorzugsweise über einen erheblichen Anteil der Länge des Behälters. Konkret ist es bevorzugt, wenn sich dieser axiale Bereich durchgehend vom proximalen Ende der Behälteröffnung bis zum distalen Behälterende erstreckt, wenn also die allmähliche Zunahme über die gesamte Länge der Behälteröffnung hin erfolgt. Es kann aber auch schon genügen, wenn die allmähliche Zunahme über einen Teil dieser Länge hinweg erfolgt, z.B. über die distalen 50% der Länge der Behälteröffnung.
Wenn beispielsweise die Behälteröffnung eine Länge von 130 mm aufweist, ist es bevorzugt, dass der mittlere Durchmesser $\langle D \rangle$ kontinuierlich um ca. 0.1 bis 3 mm über diese Länge hinweg zunimmt. Wenn ein starrer Kolben verwendet wird, beträgt die Zunahme vorzugsweise ca. 0.1 mm bis 0.5 mm, besonders bevorzugt ca. 0.3 mm, bezogen auf eine Länge von 130 mm. Wenn ein Kolben verwendet wird, der die Fähigkeit hat, sich radial aufzuweiten (z.B. ein längs geschlitzter Kolben, wie nachstehend noch näher beschrieben), beträgt die Zunahme vorzugsweise ca. 0.5 mm bis 3 mm, besonders bevorzugt ca. 1 mm, bezogen auf eine Länge von 130 mm.
Im Folgenden werden Richtungsangaben wie folgt verwendet. Der im Gehäuse verdrehbar angeordnete Behältereinsatz definiert eine zentrale Längsachse, die mit der Drehachse der Verdrehbewegung zusammenfällt. Die distale Richtung ist diejenige Richtung, in welche die Masse entlang der zentralen Längsachse zu der Gehäuseaustrittsöffnung hin vorgeschoben wird, um sie auszutragen. Die proximale Richtung bezeichnet die entsprechend entgegengesetzte Richtung dazu. Die laterale Richtung bezeichnet bezüglich der Längsachse eine radiale Richtung.
In einem zweiten Aspekt ist es eine Aufgabe der vorliegenden Erfindung, eine Austragvorrichtung zum Austragen einer schlecht fliessfähigen Masse, insbesondere eines Knochenersatzmaterials, anzugeben, die es ermöglicht, die Masse mit reduziertem Kraftaufwand aus der Vorrichtung auszutragen.
Diese Aufgabe wird durch eine Austragvorrichtung mit den Merkmalen des Anspruchs 3 gelöst.
Es wird also eine Austragvorrichtung zum Austragen einer Masse angegeben, welche aufweist:

ein Gehäuse mit einer umlaufenden Gehäusewand, einem offenen proximalen Gehäuseende und einem offenen distalen Gehäuseende, wobei eine laterale Aussparung in der Gehäusewand ausgebildet ist; und

einen im Gehäuse angeordneten Behältereinsatz mit einer umlaufenden Behälterwand, mit einem offenen proximalen Behälterende und einem distalen Behälterende, wobei in der Behälterwand eine laterale Behälteröffnung ausgebildet ist, und wobei der Behältereinsatz relativ zum Gehäuse zwischen einer Verschliessstellung, in der die Gehäusewand die laterale Behälteröffnung verschliesst, und einer Freigabestellung, in der die laterale Aussparung die laterale Behälteröffnung freigibt, um eine Längsachse verdrehbar ist.

Erfindungsgemäss erstreckt sich die laterale Behälteröffnung axial durchgehend bis zum distalen Behälterende, ohne dass sich entlang der Längsachse in distaler Richtung ein Bereich der Behälterwand an die laterale Behälteröffnung anschliesst.

Bei den Vorrichtungen des Standes der Technik war lange Zeit nicht erkannt worden, warum diese Vorrichtungen bei der Verwendung mit Knochenersatzmaterialien oder ähnlichen Massen einen sehr hohen Gegendruck aufbauen, bis hin zur völligen Blockierung. Bei der vorliegenden Erfindung wurde erkannt, dass dies mit der speziellen, sandartigen Konsistenz solcher Massen zusammenhängt. Diese Konsistenz führt dazu, dass schon geringste Verjüngungen zu einer Kompression des Materials und dadurch zu einem Zusammenbacken führen, was das weitere Austragen stark erschwert oder sogar völlig verhindert. Durch die einfache Massnahme, einen Wandbereich am distalen Ende der Behälteröffnung wegzulassen, kann eine Verjüngung des Querschnitts des Behältereinsatzes vermieden werden, und das Austragen wird ganz erheblich erleichtert.

Bevorzugt weist der durch den Behälter und das Gehäuse definierte Hohlraum insgesamt einen Querschnitt auf, der sich zumindest zwischen dem proximalen Ende der Behälteröffnung und dem distalen Behälterende nirgends verringert.

In einem dritten Aspekt ist es eine Aufgabe der vorliegenden Erfindung, eine Austragvorrichtung zum Austragen einer Masse, insbesondere eines Knochenersatzmaterials, anzugeben, welche das Befüllen mit der Masse erleichtert.

Diese Aufgabe wird durch eine Austragvorrichtung mit den Merkmalen des Anspruchs 4 gelöst.

Es wird also eine Austragvorrichtung zum Austragen einer Masse angegeben, welche aufweist:

ein Gehäuse mit einer umlaufenden Gehäusewand, einem offenen proximalen Gehäuseende und einem offenen distalen Gehäuseende, wobei eine laterale Aussparung in der Gehäusewand ausgebildet ist; und

einen im Gehäuse angeordneten Behältereinsatz mit einer umlaufenden Behälterwand, mit einem offenen proximalen Behälterende und einem distalen Behälterende, wobei in der Behälterwand eine laterale Behälteröffnung ausgebildet ist, und wobei der Behältereinsatz relativ zum Gehäuse zwischen einer Verschliessstellung, in der die Gehäusewand die laterale Behälteröffnung verschliesst, und einer Freigabestellung, in der die laterale Aussparung die laterale Behälteröffnung freigibt, um eine Längsachse verdrehbar ist.

Die Gehäusewand weist im Bereich der lateralen Aussparung eine Wandstärke (in radialer Richtung gemessen) auf, die zur lateralen Aussparung hin in Umfangsrichtung über einen Winkelbereich von mindestens 10° kontinuierlich abnimmt.

Bei einer Befüllung des Behältereinsatzes mit einer Masse, z.B. mit einem Knochenersatzmaterial, oder mit einer Komponente einer solchen Masse durch die laterale Aussparung und durch die laterale Behälteröffnung hindurch kann dadurch einerseits überschüssige Masse leicht in Umfangsrichtung weggestrichen werden, ohne dass eine Behinderung durch die Gehäusewand erfolgt, und andererseits ist dabei dennoch eine genügend grosse Stabilität der Gehäusewand gewährleistet.

Die Abnahme der radialen Wandstärke erfolgt dabei vorzugsweise nur auf der Aussenseite der Gehäusewand (d.h. auf der äusseren Gehäusemantelfläche). Sie erfolgt vorzugsweise derart, dass die Gehäusewand an der Begrenzung zur lateralen Aussparung eine klingenartige Abstreifkante bildet. Die äussere Gehäusemantelfläche und die innere äusseren Gehäusemantelfläche laufen dabei zur Abstreifkante hin in einem spitzen Winkel, vorzugweise in einem Winkel von mindestens 5° und von höchstens 60°, stärker bevorzugt mindestens 10° und höchstens 45°, aufeinander zu. Die Abstreifkante kann abgerundet sein, um die Verletzungsgefahr zu minimieren.

Der Winkelbereich, über den die Wandstärke abnimmt, beträgt vorzugsweise mindestens 20°, stärker bevorzugt mindestens 30° in Umfangsrichtung.

Eine allmähliche, kontinuierliche Abnahme der radialen Wandstärke zur lateralen Aussparung bzw. zur Abstreifkante hin stellt sicher, dass sich die Gehäusewand von der Abstreifkante aus derart nach aussen wegkrümmt, dass der Normalenvektor auf der äusseren Gehäusemantelfläche angrenzend an die Abstreifkante nirgends eine Richtungskomponente aufweist, die in Richtung einer zentral durch die laterale Aussparung verlaufenden radialen Ebene verläuft. Auf diese Weise wird beim Einfüllen eine unerwünschte Trichterwirkung, die das Abstreifen der überschüssigen Masse erschweren würde, vermieden.

Die laterale Öffnung weist vorzugsweise zwei zueinander parallele und parallel zur Längsachse verlaufende Begrenzungen auf. Die Abstreifkante verläuft in diesem Fall parallel zur Längsachse.

Auch die Behälterwand kann in Umfangsrichtung zur lateralen Behälteröffnung hin eine abnehmende radiale Wandstärke aufweisen, d.h. sie kann sich in Umfangsrichtung zur lateralen Behälteröffnung hin verjüngen. Vorzugsweise erfolgt die Abnahme der Wandstärke der Behälterwand nur innenseitig, d.h. an der inneren Behältermantelfläche. Vorzugsweise bildet die Behälterwand dadurch angrenzend zur Gehäusewand eine klingenartige Abscherkante, an die sich eine Anscherfläche anschliesst, die durch einen Teil der inneren Behältermantelfläche gebildet wird. Die äussere Behältermantelfläche und die innere Behältermantelfläche laufen dabei zur Abscherkante hin in einem spitzen Winkel, vorzugweise in einem Winkel von mindestens 5° und von höchstens 60°, stärker bevorzugt mindestens 10° und höchstens 45°, aufeinander zu. Die Abstreifkante und die Abscherkante grenzen bevorzugt in der Freigabestellung aneinander an, d.h. sie bilden gemeinsam eine Art Klinge, über die hinweg die einzufüllende Masse abgestreift werden kann.

Alle drei Aspekte der Erfindung können unabhängig voneinander verwirklicht oder beliebig miteinander kombiniert werden. Die folgenden Überlegungen gelten für alle drei Aspekte der Erfindung.

Die Aussenseite der Behälterwand liegt vorzugsweise auf der Innenseite der Gehäusewand auf und gleitet beim Verdrehen des Behältereinsatzes auf der Innenseite der Gehäusewand. Die Gehäusewand ist vorzugsweise im Bereich der lateralen Aussparung innenseitig von zylindrischer Form, und die Behälterwand ist vorzugsweise im Bereich der lateralen Behälteröffnung aussenseitig von entsprechender zylindrischer Form. Es sind aber auch andere rotationssymmetrische Formen möglich.

Bevorzugt umfasst die Austragvorrichtung ausserdem einen Kolben, der im Behältereinsatz entlang der Längsachse verschiebbar ist. Ein solcher Kolben kann aber gegebenenfalls auch durch eine externe Auspressvorrichtung bereitgestellt werden und muss daher nicht notwendig zur eigentlichen Austragvorrichtung gehören.

Der Kolben wirkt vorzugsweise nicht dichtend mit der Behälterwand zusammen. Er ist bevorzugt nicht aus einem Elastomer, sondern aus einem verhältnismässig harten Material gefertigt, das keine Dichtwirkung mit der Behälterwand erzeugt. Es ist aber auch denkbar, dass der Kolben ein weiches Material, insbesondere ein Elastomer, aufweist und/oder dass der Kolben geschlitzt ist. Insbesondere kann der Kolben derart geschlitzt sein, dass er die Fähigkeit aufweist, sich radial auszudehnen. Beispielsweise kann der Kolben einen Schlitz oder mehrere Schlitze aufweisen, welche sich parallel oder schräg zur Längsachse teilweise durch den Kolben hindurch erstrecken. Die Schlitze bilden vorzugsweise Einschnitte, die in distaler Richtung offen sind. Bevorzugt weist der Kolben zwei in distaler Richtung offene Schlitze auf, welche sich in radialer Richtung vollständig durch den Kolben hindurch erstrecken und sich kreuzen, insbesondere in einer radialen Ebene senkrecht aufeinander stehen. Durch die Schlitze wird der Kolben in mehrere Segmente unterteilt, die radial federnd mit der Stösselstange verbunden sind. Bei Einwirkung einer Kompressionskraft von aussen geben diese Segmente radial nach innen nach und verkleinern so den Aussenumfang des Kolbens. Wenn die Kompressionskraft nachlässt, federn die Segmente wieder nach aussen zurück. Ein Kolben mit einem oder mehreren derartigen Schlitzen besitzt also die Fähigkeit, sich radial ausdehnen zu können bzw. radial komprimiert zu werden.

Eine solche Fähigkeit zur radialen Ausdehnung kann beispielsweise bei einer kontinuierlichen Zunahme des freien Behälterquerschnitts bzw. des inneren Behälterdurchmessers entlang der distalen Richtung wünschenswert sein, da dann der Kolben im Bereich eines kleineren inneren Behälterdurchmessers eine kleinere radiale Ausdehnung aufweisen sollte als in einem Bereich eines grösseren inneren Behälterdurchmessers. Ein Kolben aus weichem Material würde sich mit zunehmendem innerem Behälterdurchmesser zunehmend aufweiten, und ein Kolben mit Schlitzen würde sich mit zunehmendem innerem Behälterdurchmesser zunehmend aufspreizen.

Der Kolben kann mit einer Stösselstange verbunden sein, um einen Stössel zu bilden. In diesem Fall ist es bevorzugt, wenn der Kolben radial etwas über die Stösselstange übersteht, wenn also der Kolben einen Kolbendurchmesser definiert, die Stösselstange einen Stangendurchmesser definiert, und der Kolbendurchmesser grösser ist als der Stangendurchmesser. Dadurch wird verhindert, dass Material wie z.B. Knochensplitter, welche am Kolben vorbei in den Bereich der Stösselstange gelangen, die Stösselstange blockiert. Der Kolbendurchmesser ist vorzugsweise um mindestens 0.2 mm grösser als der Stangendurchmesser, insbesondere um mindestens 0.5 mm grösser. In relativen Werten ausgedrückt, beträgt der Stangendurchmesser vorzugsweise höchstens 95% des Kolbendurchmessers.

Das Gehäuse, der Behältereinsatz, der Kolben und/oder die Stösselstange sind vorzugsweise zumindest teilweise aus Kunststoff gefertigt. Viele Kunststoffe sind weitgehend transparent für Röntgenstrahlung. Um zu ermöglichen, dass die Positionierung der Austragvorrichtung im Patientenkörper dennoch im Röntgenbild oder im CT überprüft werden kann, können das Gehäuse, der Behältereinsatz, der Kolben und/oder die Stösselstange mindestens ein röntgenopakes Funktionselement aufweisen. Diese kann insbesondere am distalen Gehäuseende, am distalen Behälterende und/oder am distalen Ende des Kolbens angeordnet sein. Insbesondere kann ein solches Funktionselement in das Gehäuse, den Behältereinsatz, den Kolben und/oder die Stösselstange eingebettet sein. Es kann sich dabei um ein Element aus einem Metall oder aus einem stark röntgenabsorbierenden Kunststoff oder Kompositmaterial handeln. Beispielsweise kann das Funktionselement ringförmig um das distale Gehäuseende, das distale Behälterende oder den Kolben herum angebracht sein.

Am distalen Gehäuseende kann ein Abweiser ausgebildet sein, welcher sich in distaler Richtung an das distale Behälterende anschliesst und entlang der distalen Richtung zur zentralen Längsachse hin gekrümmt ist. Solch ein gekrümmter Abweiser ermöglicht eine gezielte Austragung der auszutragenden Masse zu einer Seite hin.

Um ein Drehen des Behältereinsatzes im Gehäuse zu erleichtern, können ein radial abragender Haltegriff, z.B. in Form von zwei Halteflügeln, am proximalen Gehäuseende und ein radial abragender Drehgriff am offenen proximalen Behälterende ausgebildet sein.

Am Behältereinsatz kann mindestens ein Behälterdrehanschlag und am Gehäuse kann mindestens ein Gehäusedrehanschlag ausgebildet sein, wobei der Behälterdrehanschlag gegenläufig zum Gehäusedrehanschlag ausgebildet ist, und wobei bei einer Drehung des Behältereinsatzes im Gehäuse in Richtung der Freigabestellung und/oder bei einer entgegengesetzten Drehung in Richtung der Schliessstellung der Behälterdrehanschlag und der Gehäusedrehanschlag aneinander anschlagen.

Bevorzugt ist dabei der Behälterdrehanschlag als Nocken oder Steg am Aussenumfang des Behältereinsatzes ausgebildet, und der Gehäusedrehanschlag ist als komplementärer Nocken oder Steg am Innenumfang des Gehäuses ausgebildet.

Die Stösselstange kann im Bereich des distalen Stösselendes einen Stösselanschlag aufweisen, und das offene proximale Behälterende kann einen dazu komplementären Behälteranschlag aufweisen, wobei bei einem Verschieben des Stössels entlang der proximalen Richtung der Stösselanschlag derart auf den Behälteranschlag trifft, dass ein vollständiges Entfernen des Stössels aus dem Behältereinsatz behindert wird.

Bevorzugt ist der Stösselanschlag dabei als Ringwulst um die Stösselstange herum ausgebildet, und der Behälteranschlag ist als nach innen gerichtete Rastnase oder als Innenwulst am Innenumfang des Behälters ausgebildet.

Der Behältereinsatz kann mit der Masse vorbefüllt sein, und ein entfernbarer Verschluss kann die Gehäuseaustrittsöffnung verschliessen. Der Verschluss ist bevorzugt als Verschlusskappe ausgebildet, welche auf das distale Gehäuseende aufgeschoben oder auf dieses aufgeschraubt wird.

Ein erstes mögliches Verfahren zum Bereitstellen einer auszutragenden Masse, insbesondere eines Knochenersatzmaterials, in einer Austragvorrichtung der vorstehend beschriebenen Art weist die folgenden Schritte auf: i) Verdrehen des Behältereinsatzes relativ zum Gehäuse um die Längsachse, so dass die laterale Aussparung die laterale Behälteröffnung freigibt; ii) Einfüllen der auszutragenden Masse durch die laterale Aussparung und die laterale Behälteröffnung hindurch in den Behältereinsatz; und iii) Verdrehen des Behältereinsatzes relativ zum Gehäuse um die Längsachse, so dass die Gehäusewand die laterale Behälteröffnung verschliesst.

In einem zweiten möglichen Verfahren zum Bereitstellen einer auszutragenden Masse, insbesondere eines Knochenersatzmaterials, in einer Austragvorrichtung der vorstehend beschriebenen Art ist der Behältereinsatz mit einer ersten Komponente der Masse vorbefüllt, und die Gehäuseaustrittsöffnung ist durch einen Verschluss verschlossen. Das Verfahren weist dann die folgenden Schritte auf: i) Verdrehen des Behältereinsatzes relativ zum Gehäuse um die Längsachse, so dass die laterale Aussparung die laterale Behälteröffnung freigibt; ii) Zugabe einer zweiten, vorzugsweise flüssigen Komponente der Masse durch die laterale Aussparung und die laterale Behälteröffnung hindurch zur ersten Komponente; iii) Verdrehen des Behältereinsatze relativ zum Gehäuse um die Längsachse, so dass die Gehäusewand die laterale Behälteröffnung verschliesst; und iv) Entfernen des Verschlusses.

Die hier beschriebene Austragvorrichtung kann mit an sich bekannten Auspressvorrichtungen verwendet werden. Beispielsweise offenbart die WO 2013/063706 eine Auspressvorrichtung nach Art eines Pistolendispensers mit einem Adapter, wobei am Adapter eine Befestigungsstruktur zum Anbringen eines Behälters mit auszutragender Masse ausgebildet ist. Indem der Adapter und das proximale Ende der hier vorgeschlagenen Austragvorrichtung komplementär zueinander ausgebildet werden, kann die hier vorgeschlagene Austragvorrichtung ohne weiteres mit einer Auspressvorrichtung der in WO 2013/063706 offenbarten Art verwendet werden.

KURZE BESCHREIBUNG DER ZEICHNUNGEN

Bevorzugte Ausführungsformen der Erfindung werden im Folgenden anhand der Zeichnungen beschrieben, die lediglich zur Erläuterung dienen und nicht einschränkend auszulegen sind. In den Zeichnungen zeigen:

Fig. 1      eine perspektivische Explosionsdarstellung eines ersten Ausführungsbeispiels einer Austragvorrichtung mit Gehäuse, Behältereinsatz und Stössel;
Fig. 2      eine perspektivische Ansicht des Gehäuses der Figur 1;
Fig. 3      eine Seitenansicht des Gehäuses der Figur 2;
Fig. 4      einen Querschnitt durch das Gehäuse der Figur 3 in der Ebene A-A der Figur 3;
Fig. 5      eine perspektivische Ansicht des Behältereinsatzes der Figur 1;
Fig. 6      eine Seitenansicht des Behältereinsatzes der Figur 5;
Fig. 7      einen Querschnitt durch den Behältereinsatz der Figur 6 in der Ebene B-B der Figur 6;
Fig. 8      eine perspektivische Ansicht des Stössels der Figur 1;
Fig. 9      eine perspektivische Ansicht der Austragvorrichtung in einer Freigabestellung;
Fig. 10     eine Seitenansicht der Austragvorrichtung der Figur 9;

Fig. 11    einen Querschnitt durch die Austragvorrichtung der Figur 10 in der Ebene C-C der Figur 10;

Fig. 12    einen Querschnitt durch die Austragvorrichtung der Figur 10 in der Ebene D-D der Figur 10;

Fig. 13    eine perspektivische Ansicht der Austragvorrichtung in einer Verschliessstellung;

Fig. 14    eine Seitenansicht der Austragvorrichtung der Figur 13;

Fig. 15    einen Querschnitt durch die Austragvorrichtung der Figur 14 in der Ebene E-E der Figur 14;

Fig. 16    einen Querschnitt durch die Austragvorrichtung der Figur 14 in der Ebene F-F der Figur 14;

Fig. 17    eine perspektivische Ansicht der Austragvorrichtung in einem Endzustand;

Fig. 18    eine Seitenansicht der Austragvorrichtung der Figur 17;

Fig. 19    einen Querschnitt durch die Austragvorrichtung der Figur 18 in der Ebene G-G und einen Querschnitt durch die Austragvorrichtung der Figur 18 in der Ebene H-H der Figur 18;

Fig. 20    einen Querschnitt durch die Austragvorrichtung der Figur 18 in der Ebene I-I der Figur 18;

Fig. 21    eine perspektivische Ansicht eines zweiten Ausführungsbeispiels einer Austragvorrichtung;

Fig. 22    einen Querschnitt entlang einer Längsachse durch die Austragvorrichtung der Figur 21;

Fig. 23    eine perspektivische Ansicht eines dritten Ausführungsbeispiels einer Austragvorrichtung;

Fig. 24    eine Seitenansicht der Austragvorrichtung gemäss Figur 23;

Fig. 25    eine Seitenansicht eines vierten Ausführungsbeispiels einer Austragvorrichtung;

Fig. 26    einen Querschnitt entlang der Längsachse durch die Austragvorrichtung gemäss Fig. 25;

Fig. 27    eine Detailansicht des Bereichs J der Figur 26 mit arretiertem Stössel;

Fig. 28    eine Seitenansicht des Stössels der Figur 1;

Fig. 29    eine Detailansicht des Bereichs K der Figur 28;

Fig. 30    einen Querschnitt entlang der Längsachse durch die Austragvorrichtung der Figur 9;

Fig. 31    eine Detailansicht des Bereichs L der Figur 30;

Fig. 32    einen Querschnitt entlang der Längsachse durch die Austragvorrichtung der Figur 9 mit vorgeschobenem Stössel;

Fig. 33    eine Detailansicht des Bereichs M der Figur 32;

Fig. 34    eine perspektivische Ansicht eines Stössels gemäss einem fünften Ausführungsbeispiel der Erfindung;

Fig. 35    einen Querschnitt entlang der Längsachse durch eine Austragvorrichtung mit einem Stössel gemäss Figur 34;

Fig. 36    eine Detailansicht des Bereichs N der Figur 35;

Fig. 37    einen Querschnitt entlang der Längsachse durch die Austragvorrichtung der Figur 35 mit vorgeschobenem Stössel;

Fig. 38    eine Detailansicht des Bereichs O der Figur 37.

## BESCHREIBUNG BEVORZUGTER AUSFÜHRUNGSFORMEN

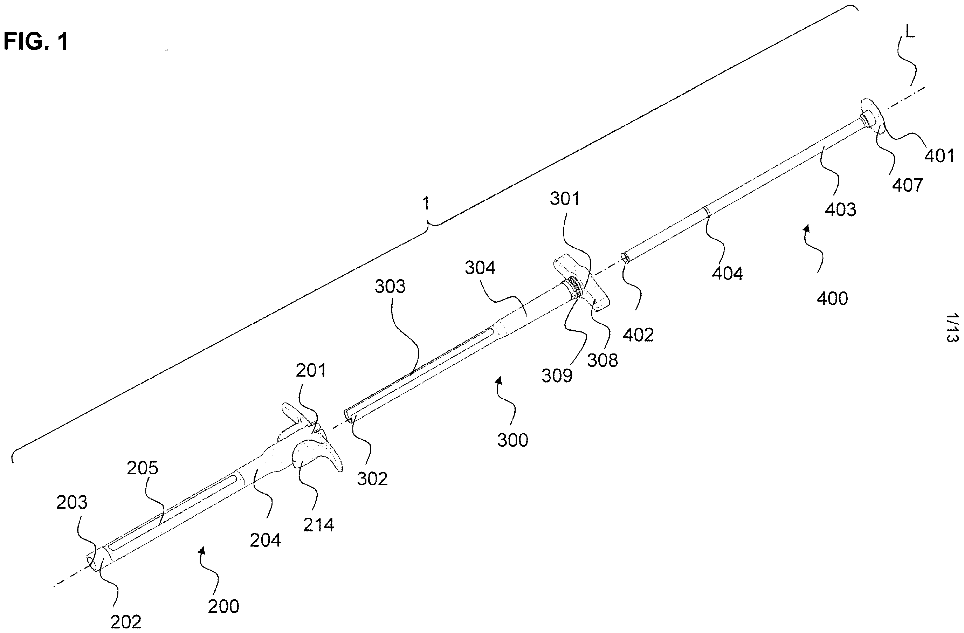

In den Figuren 1-20 ist eine erste Ausführungsform einer Austragvorrichtung zum Austragen einer schlecht fliessfähigen Masse, z.B. eines Knochenersatzmaterials, illustriert. Wie aus der Fig. 1 hervorgeht, weist die Austragvorrichtung 1 ein Gehäuse 200, einen Behältereinsatz 300 und einen Stössel 400 auf.

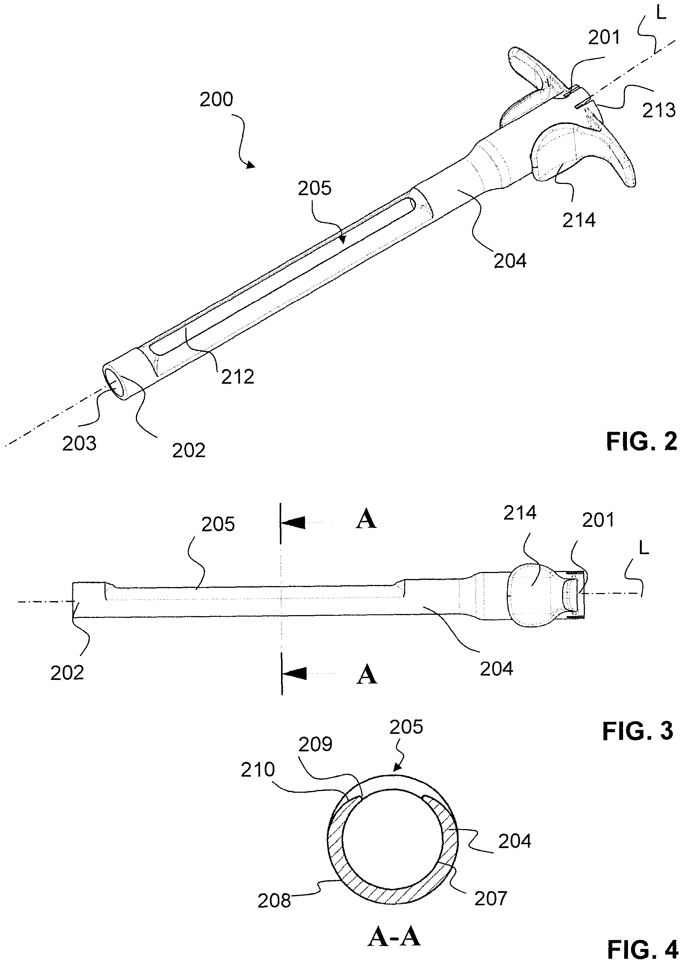

Das Gehäuse 200 ist in den Figuren 2-4 separat dargestellt. Es weist eine umlaufende Gehäusewand (Mantelwand) 204 auf, welche einen Gehäuseinnenraum 212 begrenzt und eine proximale Gehäuseeintrittsöffnung 213 und eine distale Gehäuseaustrittsöffnung 203 definiert. Das Gehäuse 200 weist im Bereich des proximalen Gehäuseendes 201 einen aufgeweiteten Bereich auf, an dem einen Haltegriff 214 in Form zweier Halteflügel ausgebildet ist. An den proximalen aufgeweiteten Bereich schliesst sich in distaler Richtung ein zylindrischer Bereich an, der sich bis zum distalen Gehäuseende 203 erstreckt. In diesem zylindrischen Bereich ist eine langgestreckte laterale Aussparung 205 (d.h. ein Fenster) in der Gehäusewand 204 ausgebildet, welche in Umfangsrichtung durch zwei sich parallel zur Längsachse L erstreckende parallele Kanten begrenzt ist.

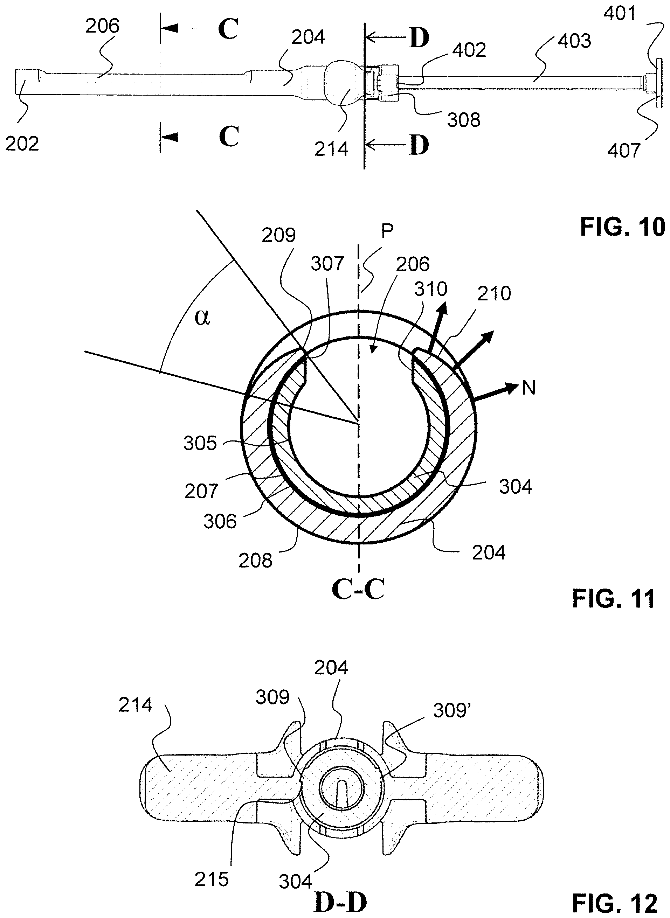

Die Gehäusewand 204 definiert eine innere Gehäusemantelfläche 207 und eine äussere Gehäusemantelfläche 208. Zur lateralen Aussparung 205 hin nimmt die Wandstärke der Gehäusewand 204 kontinuierlich in Umfangsrichtung ab. Die innere Gehäusemantelfläche 207 und die äussere Gehäusemantelfläche 208 schliessen in diesem Bereich in einer Schnittebene senkrecht zur Umfangsrichtung einen spitzen Winkel zueinander ein und treffen sich an einer Abstreifkante 209. Die Funktion dieser Abstreifkante wird nachstehend noch näher im Zusammenhang mit der Fig. 11 erläutert.

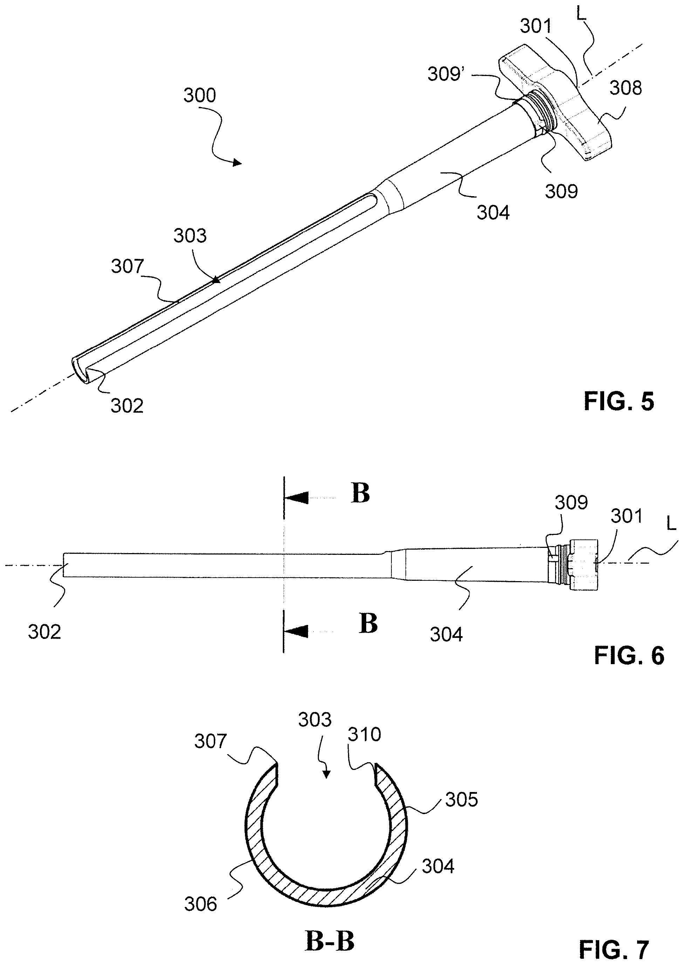

Der Behältereinsatz 300 ist in den Figuren 5-7 separat dargestellt. Er weist eine Behälterwand 304, ein offenes proximales Behälterende 301 sowie ein offenes distales Behälterende 302 auf. Die Behälterwand 304 weist proximal einen aufgeweiteten Bereich, an den sich ein schlankerer zylindrischer Bereich anschliesst. In diesem zylindrischen Bereich ist eine laterale Behälteröffnung 303 in der Behälterwand 304 ausgebildet. Die laterale Behälteröffnung 303 erstreckt sich parallel zur Längsachse L durchgehend bis zum distalen Behälterende 302, ohne dass sich entlang der Längsachse L in distaler Richtung ein Bereich der Behälterwand 304 an die laterale Behälteröffnung 303 anschliesst.

Die Behälterwand 304 definiert eine innere Behältermantelfläche 305 und eine äussere Behältermantelfläche 306. Beidseitig der lateralen Behälteröffnung sind diese Mantelflächen durch jeweils eine Abscherfläche 310 verbunden, wobei jede dieser Abscherflächen als bezüglich der Umfangsrichtung abgeschrägter Teil der inneren Behältermantelfläche 305 aufgefasst werden kann. Die Abscherflächen 310 und die äussere Behältermantelfläche 306 treffen sich an jeweils einer Abscherkante 307. Die Behälterwand 304 verjüngt sich also in Umfangsrichtung zur lateralen Behälteröffnung 303 hin derart, dass sie jeweils eine klingenartige Abscherkante 307 bildet. Dabei schliessen die jeweilige Abscherfläche 310 (als Teil der inneren Behältermantelfläche 305) und die äussere Behältermantelfläche 306 in einer Schnittebene senkrecht zur Umfangsrichtung einen spitzen Winkel zueinander ein. Die Abscherflächen 310 und Abscherkanten 307 werden im Folgenden im Zusammenhang mit der Fig. 11 näher erläutert.

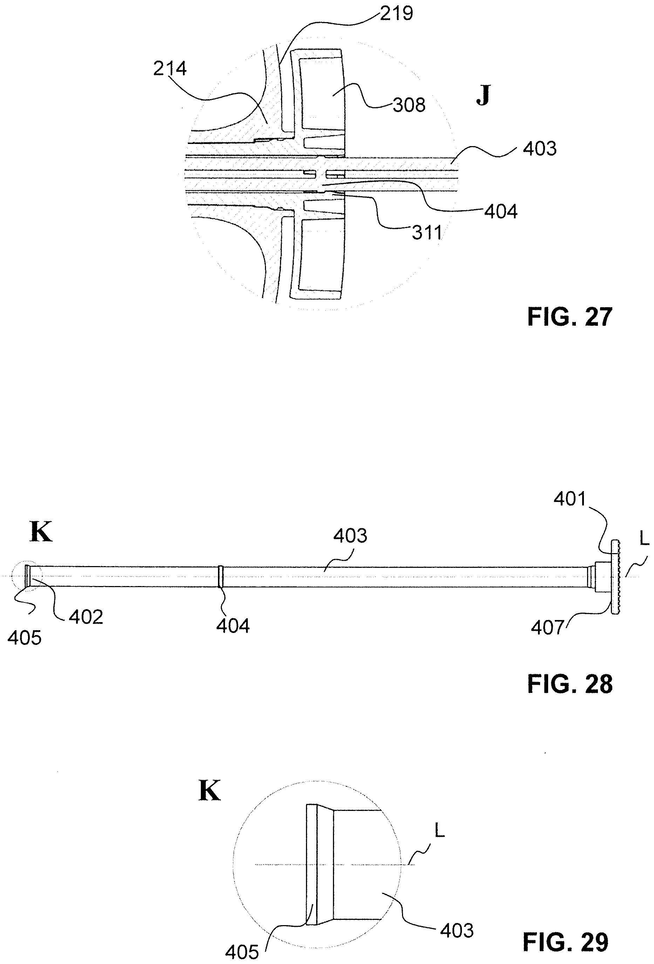

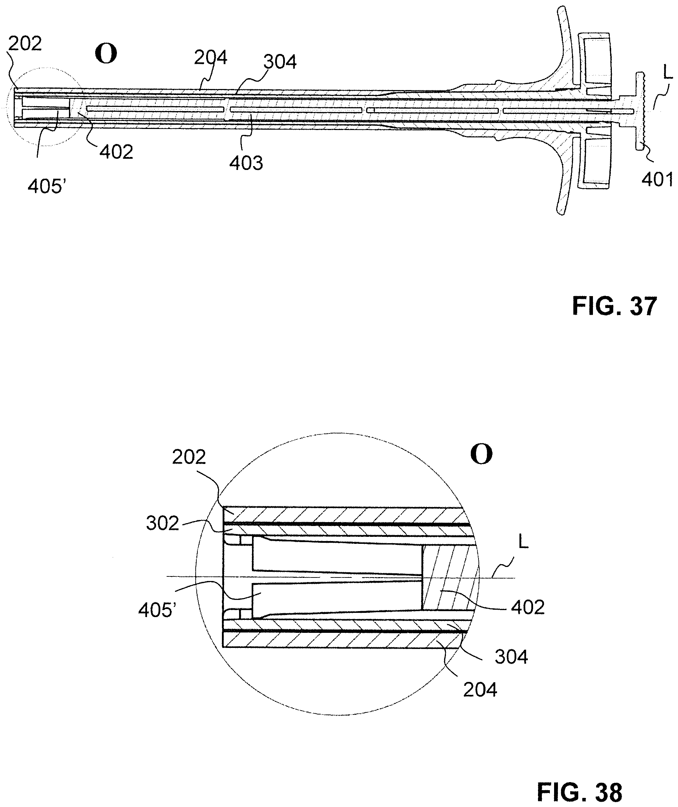

Der durch die innere Behältermantelfläche 306 begrenzte Hohlraum hat einen freien Querschnitt, der sich in distaler Richtung ganz leicht aufweitet, wie in Zusammenhang mit den Figuren 28 bis 38 noch näher erläutert wird.

Am proximalen Behälterende 301 sind zwei radial abstehenden Nocken 309, 309' ausgebildet, die als Behälterdrehanschläge wirken. Dies wird nachstehend im Zusammenhang mit der Fig. 12 noch näher erläutert. Zudem ist am proximalen Behälterende 301 ein radial abragender Drehgriff 308 ausgebildet, welcher ein Drehen des Behältereinsatzes 300 im Gehäuse 200 erleichtert.

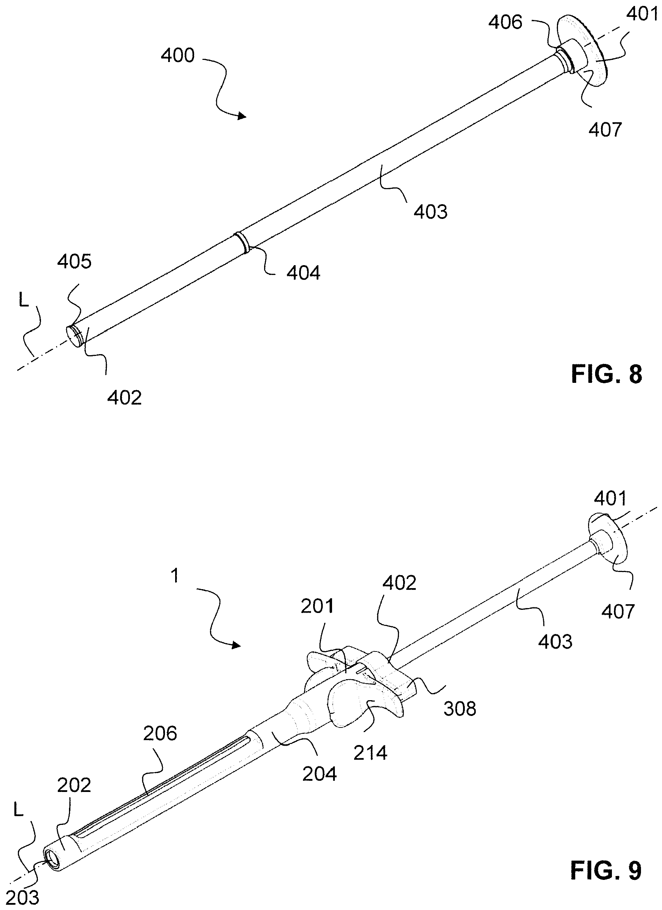

Der Stössel 400 ist in der Fig. 8 alleine dargestellt. Der Stössel 400 weist an seinem distalen Ende 402 einen Kolben 405 auf, an den sich proximal ein Stösselstange 403 anschliesst. Der Kolben 405 ragt dabei radial etwas über die Stösselstange 403 hinaus, d.h. er besitzt einen Kolbendurchmesser, welcher grösser ist als der Stösseldurchmesser der Stösselstange 403. Die sich daraus ergebenden Eigenschaften des Kolbens 405 werden in den Figuren 28 bis 33 noch genauer erläutert. An ihrem proximalen Stösselende 401 ist die Stösselstange 403 über ein Verbindungsstück 406 mit einer Druckplatte 407 verbunden. Bei der Druckplatte 407 handelt es sich in dieser Ausführungsform um eine scheibenförmige Daumenauflage, auf welche ein Benutzer manuellen Druck für die Austragung der Masse aus der Austragvorrichtung 1 ausüben kann. Die Stösselstange 403 weist einen Stösselanschlag in Form einer die Stösselstange 403 umgebenden Ringwulst 404 auf, dessen Funktion in Zusammenhang mit der Fig. 27 noch näher erläutert wird.

Die Figuren 9-12 zeigen die Austragvorrichtung in einer Freigabestellung (Befüllstellung). Dabei wurde der Behältereinsatz 300 durch das proximale Gehäuseende 201 in den Gehäuseinnenraum 212 eingeschoben, bis der Drehgriff 308 des Behältereinsatzes 300 am proximalen Gehäuseende 201 aufliegt, und der Stössel 400 wurde mit seinem distalen Stösselende 402 in das offene proximale Behälterende 301 des Behältereinsatzes 300 eingeschoben. In dieser Freigabestellung befindet sich die laterale Behälteröffnung 303 der Behälterwand 304 unmittelbar unterhalb der lateralen Aussparung 205 der Gehäusewand 204, so dass die laterale Behälteröffnung 303 zusammen mit der lateralen Aussparung 205 eine gemeinsame Befüllöffnung 206 der Austragvorrichtung 1 definieren. Die Masse wird nun von einem Benutzer in diese Befüllöffnung 206 eingefüllt, also durch die laterale Aussparung 205 des Gehäuses 200 und die laterale Behälteröffnung 303 des Behältereinsatzes 300 hindurch ins Innere des Behältereinsatzes 300 hinein. Dies kann von Hand und/oder mit Hilfe eines Werkzeugs, z.B. eines Spatels, geschehen.

Insbesondere aus Figur 11 wird ersichtlich, dass die äussere Behältermantelfläche 306 der Behälterwand 304 mit der inneren Gehäusemantelfläche 207 der Gehäusewand 204 in Kontakt steht, und dass in der Freigabestellung die Abscherkanten 307 des Behältereinsatzes unmittelbar benachbart zu den Abstreifkanten 209 des Gehäuses angeordnet sind. Zur Befüllung des Behältereinsatzes 300 kann eine Masse, z.B. ein Knochenersatzmaterial, entlang einer quer zur Längsachse L verlaufenden Abstreifrichtung über die laterale Aussparung 205 des Gehäuses 200 gestrichen werden. Dabei dient die Abstreifkante 209 als Befüllhilfe und ermöglicht ein leichteres Abstreifen der Masse. Die Masse wird durch die Abscherkante 307 nach innen hin abgeschert und gelangt so ins Innere des Behältereinsatzes 300.

Wie vorstehend schon erläutert wurde, nimmt die Wandstärke der Gehäusewand 204 zur lateralen Aussparung 205 hin aussenseitig in Umfangsrichtung über einen Winkelbereich α kontinuierlich ab, um die Abstreifkante 209 zu bilden. Dieser Winkelbereich α beträgt hier ca. 35°. Die kontinuierliche Abnahme der Wandstärke zur lateralen Aussparung 205 hin über einen relativ grossen Winkelbereich hinweg stellt sicher, dass sich die äussere Gehäusemantelfläche 208 bezüglich der Umfangsrichtung von der Abstreifkante 209 aus kontinuierlich nach aussen wegkrümmt, ohne dass dabei ein Normalenvektor N auf der äusseren Gehäusemantelfläche 208 eine Richtungskomponente aufweist, die in Richtung einer zentral durch die laterale Aussparung 205 verlaufende radiale Ebene P verläuft. Dadurch wird ein Trichtereffekt vermieden. Die radiale Ebene P stellt im vorliegenden Fall eine Symmetrieebene des Gehäuses 200 dar, und das Gehäuse 200 ist spiegelsymmetrisch bezüglich dieser radialen Ebene P ausgebildet.

In der Figur 12 ist das Zusammenwirken der radial abstehenden Nocken 309, 309', die jeweils einen Behälterdrehanschlag bilden, mit einem komplementär dazu am Gehäuse ausgebildeten Nocken 215, der einen Gehäusedrehanschlag bildet, illustriert. In der Freigabestellung schlägt der Nocken 309 an den Nocken 215 in Umfangsrichtung an und verhindert dadurch ein Weiterdrehen des Behältereinsatzes 300 im Gehäuse 200 entgegen dem Uhrzeigersinn.

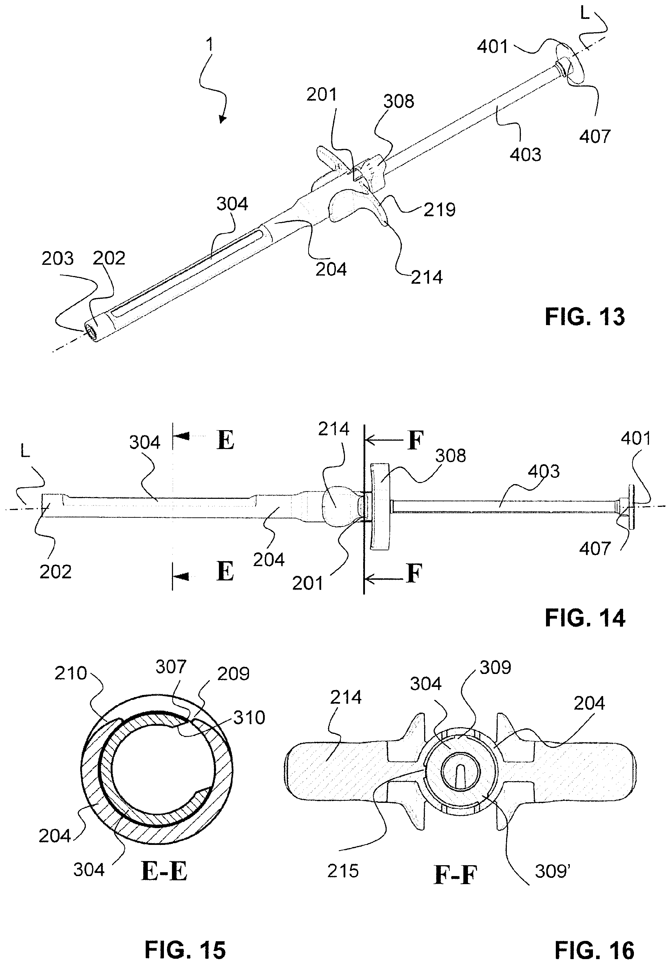

Die Figuren 13-16 illustrieren eine Zwischenstellung der Austragvorrichtung, die zwischen der Freigabestellung und der Schliessstellung liegt. In dieser Zwischenstellung wurde die Austragvorrichtung 1 zunächst in ihrer Freigabestellung mit der Masse befüllt, und der Behältereinsatz 300 wurde anschliessend im Gehäuse 200 soweit um die Längsachse L verdreht, dass die Abstreifkante 209 auf der einen Seite der lateralen Aussparung des Gehäuses mit der Abscherkante 307 auf der gegenüberliegenden Seite der lateralen Behälteröffnung in Berührung steht. Dabei bewirkt die schräg zur Umfangsrichtung verlaufende Abscherfläche 310 an der Abscherkante 307, dass Teile der Masse, welche sich noch im Bereich der Abstreifkante 209 befinden, durch die Abscherkante 307 abgeschert und in Richtung des Inneren des Behältereinsatzes verdrängt wird. Durch diese Abscherwirkung wird die Gefahr eines Verklemmens von z.B. Knochensplittern zwischen der Behälterwand 304 und der Gehäusewand 204 verringert.

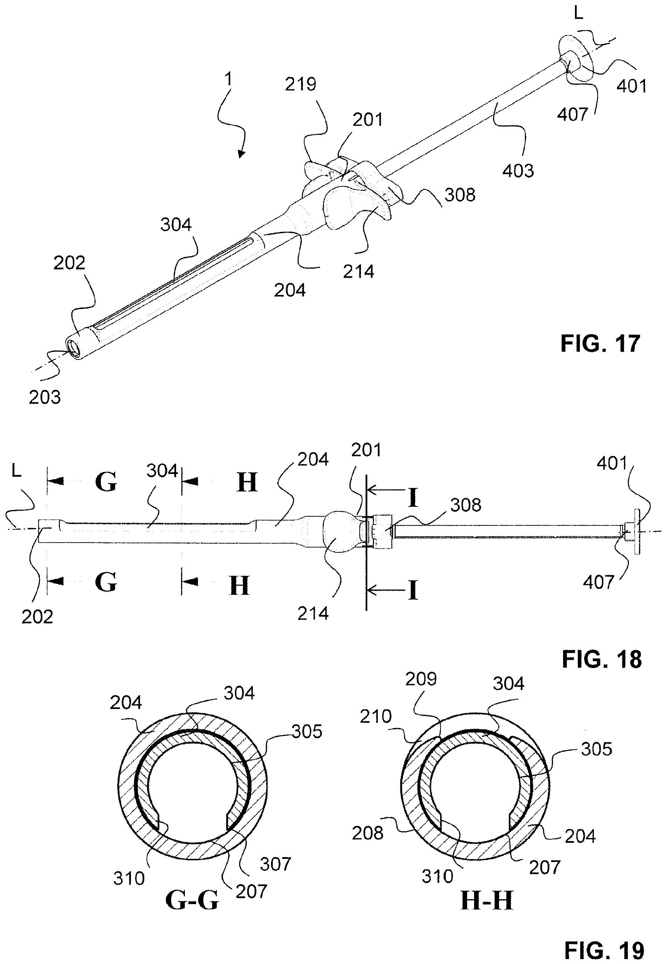

Die Figuren 17-20 illustrieren die Verschliessstellung der Austragvorrichtung. In dieser Verschliessstellung wurde der Behältereinsatz 300 ausgehend von der Zwischenstellung weiterverdreht, so dass sich die Behälteröffnung 303 des Behältereinsatzes 300 nun auf einer gegenüberliegenden Seite der lateralen Aussparung 205 des Gehäuses 200 befindet und dadurch durch die Gehäusewand 204 vollständig bedeckt wird. In anderen Worten ist nun die Befüllöffnung 206 sicher verschlossen, und die Austragvorrichtung 1 ist zum Austragen bereit.

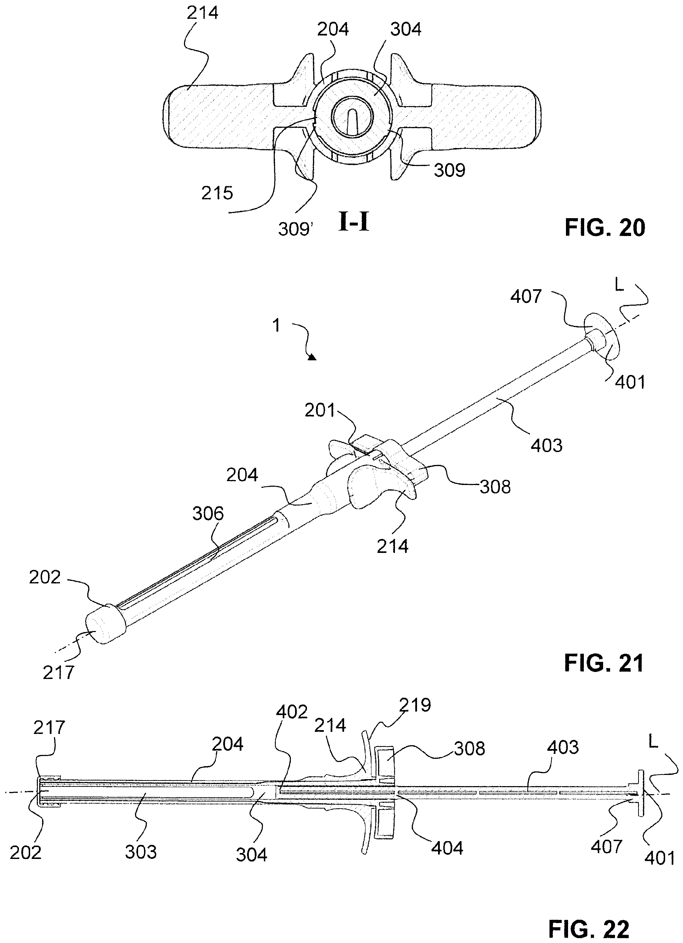

Die Fig. 20 verdeutlicht, wie in dieser Stellung der als Behälterdrehanschlag wirkende Nocken 309' in Umfangsrichtung an den als Gehäusedrehanschlag wirkenden Nocken 215 anschlägt und so das weitere Verdrehen des Behältereinsatzes 200 im Uhrzeigersinn über die Verschliessstellung hinaus verhindert.

Die Figur 21 zeigt eine Austragsvorrichtung gemäss einer zweiten Ausführungsform. Diese zweite Ausführungsform entspricht weitgehend der ersten Ausführungsform, weist aber einen entfernbaren Verschluss 217 in Form einer Verschlusskappe auf. Die Verschlusskappe 217 ist hier als Schraubkappe ausgebildet. Sie verschliesst die Gehäuseaustrittsöffnung 203, indem ein Innengewinde, welches auf einer Innenseite der Verschlusskappe ausgebildet ist, mit einem entsprechenden Aussengewinde, das an der äusseren Gehäusemantelfläche 208 am distalen Gehäuseende 202 ausgebildet ist, in Gewindeeingriff steht.

Diese Ausführungsform ist vor allem dann vorteilhaft, wenn der Behältereinsatz 300 mit einer Komponente der auszutragenden Masse (z.B. einem Pulver) vorbefüllt in das Gehäuse 200 eingeschoben wurde. In der Verschliessstellung ist diese Komponente vollständig in der Austragvorrichtung eingeschlossen. Um nun eine weitere Komponente (z.B. eine flüssige Komponente wie Blut, Blutplasma oder Knochenmarkaspirat) hinzuzufügen, ergreift ein Benutzer den Drehgriff 308 des Behältereinsatzes 300 und verdreht diesen derart, dass die Austragvorrichtung in die Freigabestellung gelangt. Der Benutzer kann nun die zweite Komponente einfüllen (z.B. das Pulver benetzen) und die Austragvorrichtung 1 durch Verdrehen des Behältereinsatzes 300 im Gehäuse 200 erneut in die Verschliessstellung überführen. Durch Entfernen der Verschlusskappe 217 wird anschliessend die Gehäuseaustrittsöffnung 203 freigegeben, so dass durch Vorschieben des Stössels 400 die derart bereitgestellte Masse ausgetragen werden kann.

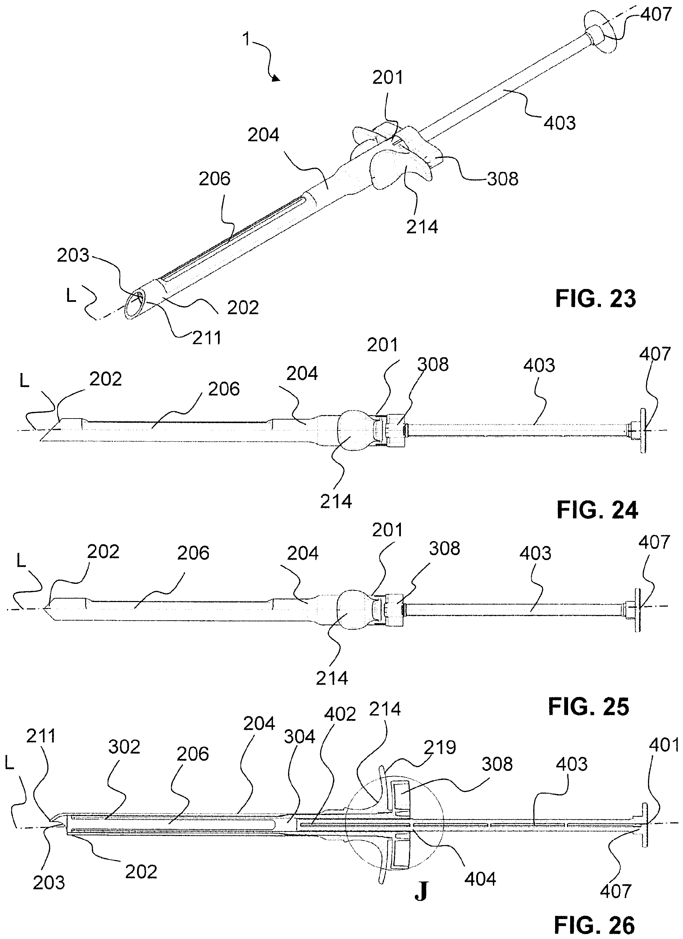

Die Figuren 23 bis 26 zeigen eine dritte und eine vierte Ausführungsform einer Austragvorrichtung. Diese entsprechen wiederum weitgehend der ersten Ausführungsform, wobei jedoch das distale Gehäuseende 202 jeweils unterschiedlich ausgebildet ist. So verläuft in der dritten Ausführungsform der Figuren 23 und 24 das distale Gehäuseende schräg zur Längsachse L. In der vierten Ausführungsform der Figuren 25 und 25 schliesst sich an das distale Gehäuseende ein Abweiser 211 an, welcher in distaler Richtung entlang der distalen Richtung zur zentralen Längsachse L hin gekrümmt ist. Durch diese Ausführungsformen wird es erleichtert, die Masse zu einer Seite hin auszutragen.

Figur 27 zeigt eine Detailansicht des Bereichs J der Figur 26, wobei der Stössel 400 mit seinem Stösselanschlag 404 in Form eines Ringwulsts an einem Behälteranschlag 311 in Form einer Rastnase im offenen proximalen Behälterende 301 derart anschlägt, dass ein Verschieben des Stössels 400 entlang der proximalen Richtung durch das proximale Gehäuseende 201 aus dem Behältereinsatz 300 heraus verhindert ist. Dadurch kann ein Herausfallen des Stössels 400 aus der Austragvorrichtung heraus verhindert werden.

Die Figuren 28 und 29 illustrieren den Kolben 400 gemäss der ersten Ausführungsform, wobei nun insbesondere die Eigenschaften des radial über die Stösselstange 403 vorstehenden Kolbens 405 verdeutlicht werden sollen. Der Kolben 405 definiert einen Kolbendurchmesser, während die Stösselstange 403 einen kleineren Stangendurchmesser definiert. Der Übergang zwischen Kolben und Stösselstange erfolgt über eine konische Rampe.

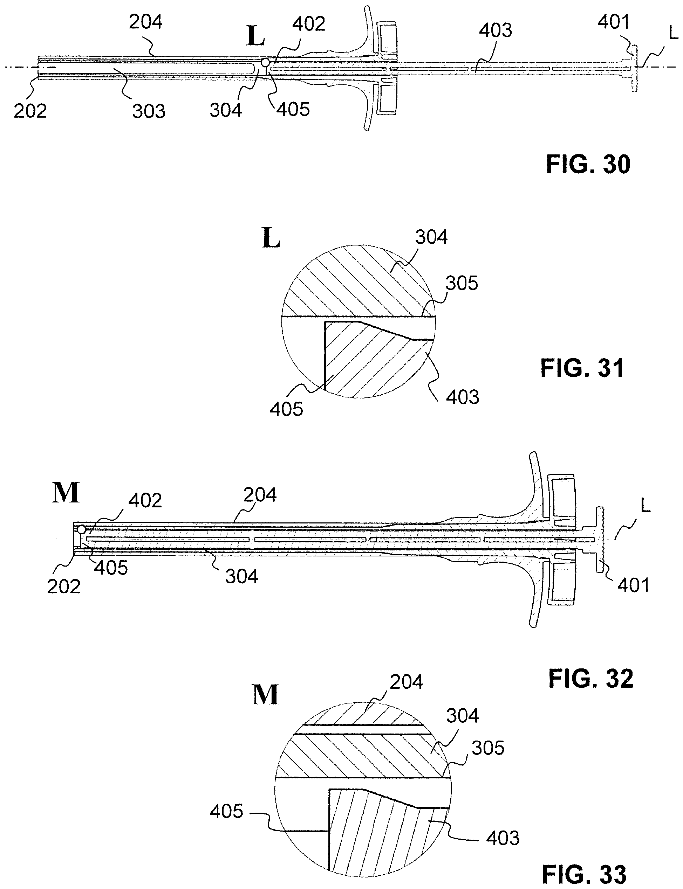

Die Figuren 30 bis 33 illustrieren eine Austragvorrichtung 1 gemäss der ersten Ausführungsform, deren Behältereinsatz 300 sich innenseitig in die distale Richtung kontinuierlich aufweitet. Sowohl die äussere Behältermantelfläche 306 als auch die Stösselstange 403 haben eine zylindrische Form mit jeweils einem konstanten Durchmesser, während die innere Behältermantelfläche 305 einen inneren Behälterdurchmesser definiert, welcher entlang der Längsachse L in distaler Richtung kontinuierlich zunimmt. Mit zunehmendem Vorschub des Stössels 400 im Behältereinsatz 300 wird daher die radiale Beabstandung der Stösselstange 403 von der inneren Behältermantelfläche 305 zunehmend grösser.

Die Figur 30 zeigt die Austragvorrichtung 1 mit darin aufgenommenem Stössel 400, dessen distales Stösselende 402 sich im Bereich des proximalen Behälterendes 302 befindet. Die Figur 31 zeigt eine Detailansicht des Bereichs L der Figur 30, woraus erkennbar wird, dass sich dabei die äussere Umfangsfläche des Kolbens 405 in unmittelbarer Nähe zu der inneren Behältermantelfläche 305 befindet, dazwischen jedoch ein schmaler Spalt ausgebildet ist. Daran schliesst sich in proximaler Richtung ein grösserer Spalt zwischen der äusseren Mantelfläche der Stösselstange 403 und der inneren Behältermantelfläche 305 an. Die Figuren 32 und 33 zeigen die Austragvorrichtung 1 mit darin aufgenommenem Stössel 400, wobei der Stössel 400 in distaler Richtung vorgeschoben wurde und sich nun mit seinem distalen Stösselende 402 im Bereich des distalen Gehäuseendes 202 und Behälterendes 302 befindet. Da die innere Behältermantelfläche 305 einen Behälterdurchmesser definiert, welcher sich entlang der distalen Richtung zunehmend vergrössert, nimmt auch der Spalt zwischen der äusseren Mantelfläche des Kolbens 405 bzw. der äusseren Mantelfläche der Stösselstange 403 und der inneren Behältermantelfläche 305 entsprechend zu. Durch diese in distaler Richtung zunehmenden Spalte kann ein Verklemmen des Stössels 400 vermieden werden, falls die Masse in den Spalt zwischen dem Kolben 405 und der inneren Behältermantelfläche 305 oder sogar am Kolben 405 vorbei in den Bereich der Stösselstange 403 gelangt. Dabei genügt es, wenn sich der Durchmesser des Behälters nur ein ganz wenig aufweitet, z.B. um weniger als einen Zehntel eines Millimeters pro Zentimeter Länge (d.h. um weniger als 1 Prozent).

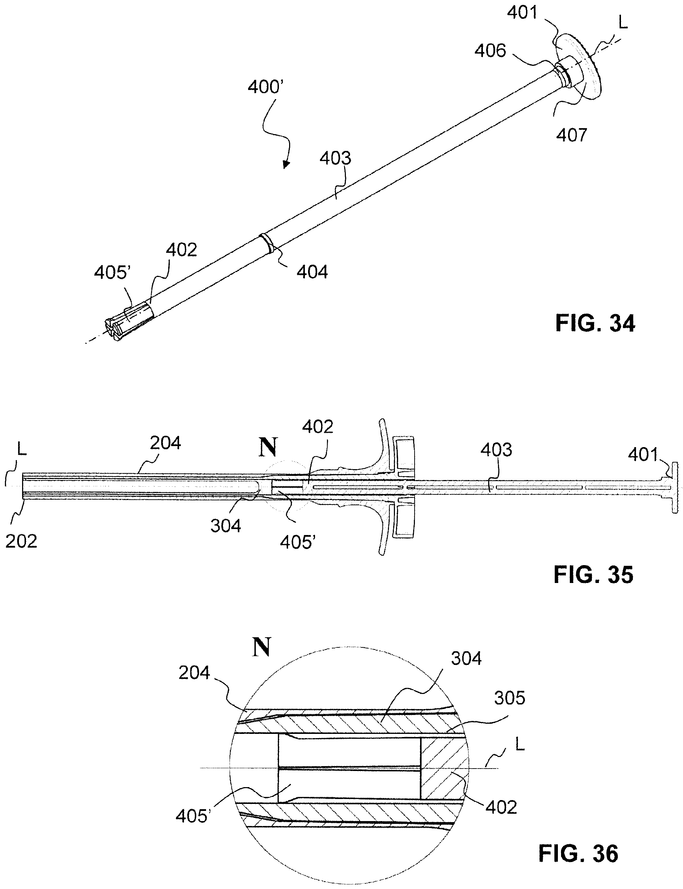

Eine fünfte Ausführungsform der Erfindung ist in den Figuren 34 bis 38 illustriert. Diese Ausführungsform entspricht der ersten Ausführungsform; jedoch weist der Stössel 400' an seinem distalen Stösselende einen Kolben 405' mit zwei sich kreuzenden Schlitzen (Einschnitten) auf, wobei sich die Schlitze radial durch den Kolben 405' hindurch erstrecken und in die distale Richtung offen sind. Diese Schlitze stehen dabei senkrecht aufeinander und unterteilen den Kolben in vier gleiche Segmente. Diese Segmente können radial nach innen, in Richtung der Längsachse, einfedern, wenn eine radiale Kraft auf sie einwirkt. Dies ermöglicht, dass sich der Kolben 405' entlang der radialen Richtung radial ausdehnt respektive radial komprimiert, wenn er durch einen Behälter mit veränderlichem freien Querschnitt geführt wird.

Dazu illustrieren die Figuren 35 bis 38, analog zu den Figuren 30 bis 33, die Verwendung dieses Stössels 400' in einer Austragvorrichtung 1, deren Behältereinsatz 300 sich innenseitig in die distale Richtung kontinuierlich konisch aufweitet. Der geschlitzte Kolben 405' weist dabei im proximalen Bereich des Behältereinsatzes 300, mit kleinerem inneren Behälterdurchmesser, eine kleinere radiale Ausdehnung und einen kleineren Umfang auf als im distalen Bereich mit grösserem inneren Behälterdurchmesser, da sich mit zunehmendem inneren Behälterdurchmesser die Segmente des Kolbens 405' zunehmend federnd aufspreizen. Der geschlitzte Kolben 405' gewährleistet dabei, dass die auszutragende Masse auch bei einem sich relativ stark distal aufweitenden Behältereinsatz 300 dennoch effizient und vollständig ausgetragen werden kann.

## BEZUGSZEICHENLISTE

| 1 | Austragvorrichtung | 303 304 | laterale Behälteröffnung Behälterwand |
|---|---|---|---|
| 200 | Gehäuse | 305 | innere Behältermantelfläche |
| 201 | proximales Gehäuseende | 306 | äussere Behältermantelfläche |
| 202 | distales Gehäuseende | 307 | Abscherkante |
| 203 | Gehäuseaustrittsöffnung | 308 | Drehgriff |
| 204 | Gehäusewand | 309, 309' | Behälterdrehanschlag |
| 205 | laterale Aussparung | 310 | Abscherfläche |
| 206 | Befüllöffnung | 311 | Behälteranschlag |
| 207 | innere Gehäusemantelfläche | | |
| 208 | äussere Gehäusemantelfläche | 400,400' | Stössel |
| 209 | Abstreifkante | 401 | proximales Stösselende |
| 210 | Abstreiffläche | 402 | distales Stösselende |
| 211 | Abweiser | 403 | Stösselstange |
| 212 | Gehäuseinnenraum | 404 | Stösselanschlag |
| 213 | Gehäuseeintrittsöffnung | 405,405' | Kolben |
| 214 | Haltegriff | 406 | Verbindungsstück |
| 215 | Gehäusedrehanschlag | 407 | Druckplatte |
| 216 | Griffoberfläche | | |
| 217 | Verschluss | 500 | Funktionselement |
| 300 | Behältereinsatz | L | Längsachse |
| 301 | proximales Behälterende | P | radiale Ebene |
| 302 | distales Behälterende | N | Normalenvektor |

## Patentansprüche

1. Austragvorrichtung zum Austragen einer Masse, aufweisend:

   ein Gehäuse (200) mit einer umlaufenden Gehäusewand (204), einem offenen proximalen Gehäuseende (201) und einem offenen distalen Gehäuseende (202), wobei eine laterale Aussparung (205) in der Gehäusewand (204) ausgebildet ist; und
   einen im Gehäuse (200) angeordneten Behältereinsatz (300) mit einer umlaufenden Behälterwand (304), mit einem offenen proximalen Behälterende (301) und einem distalen Behälterende (302), wobei in der Behälterwand (304) eine laterale Behälteröffnung (303) ausgebildet ist, und wobei der Behältereinsatz (300) relativ zum Gehäuse (200) zwischen einer Verschliessstellung, in der die Gehäusewand (204) die laterale Behälteröffnung (303) verschliesst, und einer Freigabestellung, in der die laterale Aussparung (205) die laterale Behälteröffnung (303) freigibt, um eine Längsachse (L) verdrehbar ist,

**dadurch gekennzeichnet, dass** der Behältereinsatz (300) einen freien Behälterquerschnitt aufweist, der entlang der Längsachse (L) über einen axialen Bereich in distaler Richtung kontinuierlich zunimmt.

2. Austragvorrichtung nach Anspruch 1, wobei sich der axiale Bereich, über den der Behälterquerschnitt kontinuierlich zunimmt, von einem proximalen Ende der Behälteröffnung (303) bis zum distalen Behälterende (302) erstreckt.

3. Austragvorrichtung zum Austragen einer Masse, aufweisend:

ein Gehäuse (200) mit einer umlaufenden Gehäusewand (204), einem offenen proximalen Gehäuseende (201) und einem offenen distalen Gehäuseende (202), wobei eine laterale Aussparung (205) in der Gehäusewand (204) ausgebildet ist; und
einen im Gehäuse (200) angeordneten Behältereinsatz (300) mit einer umlaufenden Behälterwand (304), mit einem offenen proximalen Behälterende (301) und einem distalen Behälterende (302), wobei in der Behälterwand (304) eine laterale Behälteröffnung (303) ausgebildet ist, und wobei der Behältereinsatz (300) relativ zum Gehäuse (200) zwischen einer Verschliessstellung, in der die Gehäusewand (204) die laterale Behälteröffnung (303) verschliesst, und einer Freigabestellung, in der die laterale Aussparung (205) die laterale Behälteröffnung (303) freigibt, um eine Längsachse (L) verdrehbar ist,
**dadurch gekennzeichnet, dass** sich die laterale Behälteröffnung (303) axial durchgehend bis zum distalen Behälterende (302) erstreckt, ohne dass sich entlang der Längsachse (L) in distaler Richtung ein Bereich der Behälterwand (304) an die laterale Behälteröffnung (303) anschliesst.

4. Austragvorrichtung zum Austragen einer Masse, aufweisend:

ein Gehäuse (200) mit einer umlaufenden Gehäusewand (204), einem offenen proximalen Gehäuseende (201) und einem offenen distalen Gehäuseende (202), wobei eine laterale Aussparung (205) in der Gehäusewand (204) ausgebildet ist;
einen im Gehäuse (200) angeordneten Behältereinsatz (300) mit einer umlaufenden Behälterwand (304), mit einem offenen proximalen Behälterende (301) und einem distalen Behälterende (302), wobei in der Behälterwand (304) eine laterale Behälteröffnung (303) ausgebildet ist, und wobei der Behältereinsatz (300) relativ zum Gehäuse (200) zwischen einer Verschliessstellung, in der die Gehäusewand (204) die laterale Behälteröffnung (303) verschliesst, und einer Freigabestellung, in der die laterale Aussparung (205) die laterale Behälteröffnung (303) freigibt, um eine Längsachse (L) verdrehbar ist,
**dadurch gekennzeichnet, dass** die Gehäusewand (204) im Bereich der lateralen Aussparung (205) eine Wandstärke aufweist, die zur lateralen Aussparung (205) hin in Umfangsrichtung über einen Winkelbereich (a), der mindestens 10° beträgt, kontinuierlich abnimmt.

5. Austragvorrichtung gemäss Anspruch 4, wobei die Wandstärke der Gehäusewand (204) in Umfangsrichtung zur lateralen Aussparung (205) hin derart abnimmt, dass die Gehäusewand (204) an der lateralen Aussparung (205) eine Abstreifkante (209) bildet.

6. Austragvorrichtung gemäss Anspruch 5, wobei sich die Behälterwand (304) in Umfangsrichtung zur lateralen Behälteröffnung (303) hin derart verjüngt, dass sie angrenzend zur Gehäusewand (204) eine klingenartige Abscherkante (307) bildet, und wobei die Abstreifkante (209) und die Abscherkante (307) in der Freigabestellung aneinander angrenzen.

7. Austragvorrichtung nach einem der vorhergehenden Ansprüche, welche ausserdem einen Kolben (405, 405') umfasst, der im Behältereinsatz (305) entlang der Längsachse (L) verschiebbar ist.

8. Austragvorrichtung nach Anspruch 7,

wobei der Kolben (405, 405') einen Kolbendurchmesser definiert,
wobei der Kolben (405, 405') mit einer Stösselstange (403) verbunden ist,
wobei die Stösselstange (403) einen Stangendurchmesser definiert, und

wobei der Kolbendurchmesser grösser ist als der Stangendurchmesser.

9. Austragvorrichtung nach Anspruch 7 oder 8, **dadurch gekennzeichnet, dass** der Kolben (405') derart geschlitzt ist, dass er die Fähigkeit aufweist, sich radial auszudehnen.

10. Austragvorrichtung (1) gemäss einem der vorhergehenden Ansprüche, wobei am distalen Gehäuseende (202) ein Abweiser (211) ausgebildet ist, der sich in distaler Richtung an das distale Behälterende (302) anschliesst und entlang der distalen Richtung zur zentralen Längsachse (L) hin gekrümmt ist.

11. Austragvorrichtung (1) gemäss einem der vorhergehenden Ansprüche, wobei am Behältereinsatz (300) mindestens ein Behälterdrehanschlag (309, 309') und am Gehäuse (200) mindestens ein Gehäusedrehanschlag (215) ausgebildet sind, wobei der Behälterdrehanschlag (309, 309') gegenläufig zum Gehäusedrehanschlag (215) ausgebildet ist, und wobei der Behälterdrehanschlag (309, 309') und der Gehäusedrehanschlag (215) derart angeordnet sind, dass der Behälterdrehanschlag (309, 309') und der Gehäusedrehanschlag (215) bei einer Drehung des Behältereinsatzes (300) im Gehäuse (200) in Richtung der Freigabestellung und/oder bei einer entgegengesetzten Drehung in Richtung der Schliessstellung aneinander anschlagen.

12. Austragvorrichtung (1) gemäss einem der vorhergehenden Ansprüche, wobei der Behältereinsatz (300) mit einer Masse vorbefüllt ist, und wobei die Austragvorrichtung einen entfernbaren Verschluss (217) aufweist, der die Gehäuseaustrittsöffnung (203) verschliesst.

13. Verfahren zum Bereitstellen einer auszutragenden Masse in einer Austragvorrichtung (1), welche ein Gehäuse (200) mit einer Gehäusewand (204) und einer darin ausgebildeten lateralen Aussparung (205) und einen im Gehäuse (200) verdrehbar angeordneten Behältereinsatz (300) mit einer Behälterwand (204) und einer darin ausgebildeten lateralen Behälteröffnung (303) aufweist, wobei der Behältereinsatz (300) mit einer ersten Komponente der Masse vorbefüllt ist und ein entfernbarer Verschluss (217) die Austragvorrichtung (1) verschliesst, und wobei das Verfahren die folgenden Schritte aufweist:

Verdrehen des Behältereinsatzes (300) relativ zum Gehäuse (200) um die Längsachse (L), so dass die laterale Aussparung (205) die laterale Behälteröffnung (303) freigibt;
Zugabe einer zweiten Komponente der Masse durch die laterale Aussparung (205) und die laterale Behälteröffnung (303) hindurch zur ersten Komponente im Behältereinsatz (300);
Verdrehen des Behältereinsatzes (300) relativ zum Gehäuse (200) um die Längsachse (L), so dass die Gehäusewand (204) die laterale Behälteröffnung (303) verschliesst; und
Entfernen des Verschlusses (217).

FIG. 1

**FIG. 2**

**FIG. 3**

A-A

**FIG. 4**

300

301

L

309'

308

309

304

303

307

302

**FIG. 5**

B

309

301

L

B

302

304

**FIG. 6**

303

310

307

305

306

304

**B-B**

**FIG. 7**

FIG. 8

FIG. 9

FIG. 10

C-C

FIG. 11

D-D

FIG. 12

**FIG. 13**

**FIG. 14**

**E-E**

**FIG. 15**

**F-F**

**FIG. 16**

**FIG. 17**

**FIG. 18**

**FIG. 19**

FIG. 20

**I-I**

FIG. 21

FIG. 22

FIG. 23

FIG. 24

FIG. 25

FIG. 26

219

214

308

J

403

404

311

**FIG. 27**

401

K

403

L

402

404

407

405

**FIG. 28**

K

L

405

403

**FIG. 29**

**FIG. 30**

**FIG. 31**

**FIG. 32**

**FIG. 33**

**FIG. 34**

**FIG. 35**

**FIG. 36**

FIG. 37

FIG. 38

# EP 3 597 130 A1

Europäisches Patentamt
European Patent Office
Office européen des brevets

## EUROPÄISCHER RECHERCHENBERICHT

Nummer der Anmeldung

EP 19 19 6674

### EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| Y,D | US 6 582 438 B2 (DEMAYO EDWARD [US]) 24. Juni 2003 (2003-06-24) | 3 | INV. A61B17/88 |
| A | * das ganze Dokument * ----- | 1,2,4 | A61F2/46 A61B17/70 |
| Y | US 4 784 607 A (FRANCOIS MICHEL [FR]) 15. November 1988 (1988-11-15) | 3 | A61C9/00 |
| A | * Spalte 1, Zeilen 5-41 * * Spalte 2, Zeile 26 - Spalte 3, Zeile 42 * | 1,2 | |
| | * Abbildungen 1-6 * ----- | | |
| A | US 6 309 372 B1 (FISCHER DAN E [US] ET AL) 30. Oktober 2001 (2001-10-30) * Spalte 5, Zeile 1 - Spalte 8, Zeile 11 * * Abbildungen 1-5c * ----- | 1,2 | |
| A | US 2010/121268 A1 (KELLER WILHELM A [CH]) 13. Mai 2010 (2010-05-13) * Seite 1, Absatz 18 - Seite 2, Absatz 23 * | 1,2 | |
| | * Abbildung 5 * ----- | | RECHERCHIERTE SACHGEBIETE (IPC) |
| A | CH 708 198 A1 (MEDMIX SYSTEMS AG [CH]) 15. Dezember 2014 (2014-12-15) * Seite 5, Absatz 37-40 * * Abbildungen 1, 2 * ----- | 1,2 | A61B A61F A61C A61M |
| A | US 5 697 903 A (FISCHER DAN E [US]) 16. Dezember 1997 (1997-12-16) * Spalte 4, Zeile 27 - Spalte 5, Zeile 40 * | 1,2 | |
| | * Abbildungen 1-4 * ----- -/-- | | |

Der vorliegende Recherchenbericht wurde für alle Patentansprüche erstellt

| Recherchenort | Abschlußdatum der Recherche | Prüfer |
|---|---|---|
| Berlin | 25. Oktober 2019 | Tamme, H |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

........................................................

& : Mitglied der gleichen Patentfamilie, übereinstimmendes Dokument

EPO FORM 1503 03.82 (P04C03)

Seite 1 von 2

**Europäisches Patentamt**

**European Patent Office**

**Office européen des brevets**

## EUROPÄISCHER RECHERCHENBERICHT

**Nummer der Anmeldung**

EP 19 19 6674

### EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| A | WO 2008/014622 A1 (0696578 B C LTD INC BC0696578 [CA]; CLEATOR IAIN G M [CA]; MORIN MARC) 7. Februar 2008 (2008-02-07) * Seite 4, Absatz 23 - Seite 5, Absatz 24 * <br> * Seite 7, Absatz 33 - Seite 8, Absatz 34 * <br> * Abbildungen 1g-1h, 4a-5c * <br> ----- | 1,2 | |
| A | US 2014/303744 A1 (EVANS DOUGLAS G [US] ET AL) 9. Oktober 2014 (2014-10-09) * Seite 12, Absatz 120-121 * <br> * Abbildung 1 * <br> ----- | 1-13 | |
| A | EP 2 774 583 A1 (EBI LLC [US]) 10. September 2014 (2014-09-10) * Spalte 4, Absatz 18 - Spalte 5, Absatz 21 * <br> * Abbildungen 1-2 * <br> ----- | 1-13 | |

**RECHERCHIERTE SACHGEBIETE (IPC)**

Der vorliegende Recherchenbericht wurde für alle Patentansprüche erstellt

| Recherchenort | Abschlußdatum der Recherche | Prüfer |
|---|---|---|
| Berlin | 25. Oktober 2019 | Tamme, H |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument
...........................................................................
& : Mitglied der gleichen Patentfamilie, übereinstimmendes Dokument

EPO FORM 1503 03.82 (P04C03)

**ANHANG ZUM EUROPÄISCHEN RECHERCHENBERICHT
ÜBER DIE EUROPÄISCHE PATENTANMELDUNG NR.**

EP 19 19 6674

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

25-10-2019

| Im Recherchenbericht angeführtes Patentdokument | Datum der Veröffentlichung | Mitglied(er) der Patentfamilie | Datum der Veröffentlichung |
|---|---|---|---|
| US 6582438 B2 | 24-06-2003 | KEINE | |
| US 4784607 A | 15-11-1988 | DE 3678910 D1 | 29-05-1991 |
| | | EP 0225265 A1 | 10-06-1987 |
| | | ES 2021606 B3 | 16-11-1991 |
| | | FR 2590487 A1 | 29-05-1987 |
| | | US 4784607 A | 15-11-1988 |
| US 6309372 B1 | 30-10-2001 | AU 6341400 A | 05-02-2001 |
| | | US 6309372 B1 | 30-10-2001 |
| | | WO 0105321 A2 | 25-01-2001 |
| US 2010121268 A1 | 13-05-2010 | CN 101678386 A | 24-03-2010 |
| | | EP 2152435 A1 | 17-02-2010 |
| | | JP 5166521 B2 | 21-03-2013 |
| | | JP 2010531276 A | 24-09-2010 |
| | | US 2010121268 A1 | 13-05-2010 |
| | | WO 2008151456 A1 | 18-12-2008 |
| CH 708198 A1 | 15-12-2014 | KEINE | |
| US 5697903 A | 16-12-1997 | AU 1969997 A | 10-09-1997 |
| | | DE 19781608 T1 | 01-04-1999 |
| | | EP 0964719 A1 | 22-12-1999 |
| | | US 5697903 A | 16-12-1997 |
| | | WO 9730751 A1 | 28-08-1997 |
| WO 2008014622 A1 | 07-02-2008 | CA 2660076 A1 | 07-02-2008 |
| | | US 2008097286 A1 | 24-04-2008 |
| | | US 2008300575 A1 | 04-12-2008 |
| | | WO 2008014622 A1 | 07-02-2008 |
| US 2014303744 A1 | 09-10-2014 | AU 2003243711 A1 | 28-01-2005 |
| | | CA 2503904 A1 | 20-01-2005 |
| | | EP 1648347 A1 | 26-04-2006 |
| | | EP 2305180 A1 | 06-04-2011 |
| | | JP 4635276 B2 | 23-02-2011 |
| | | JP 2006527009 A | 30-11-2006 |
| | | US 2003236573 A1 | 25-12-2003 |
| | | US 2004034434 A1 | 19-02-2004 |
| | | US 2004064193 A1 | 01-04-2004 |
| | | US 2004127987 A1 | 01-07-2004 |
| | | US 2004138758 A1 | 15-07-2004 |
| | | US 2008015709 A1 | 17-01-2008 |
| | | US 2009030528 A1 | 29-01-2009 |
| | | US 2009043400 A1 | 12-02-2009 |

Für nähere Einzelheiten zu diesem Anhang : siehe Amtsblatt des Europäischen Patentamts, Nr.12/82

EPO FORM P0461

Seite 1 von 2

**ANHANG ZUM EUROPÄISCHEN RECHERCHENBERICHT
ÜBER DIE EUROPÄISCHE PATENTANMELDUNG NR.**

EP 19 19 6674

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

25-10-2019

| Im Recherchenbericht angeführtes Patentdokument | Datum der Veröffentlichung | Mitglied(er) der Patentfamilie | Datum der Veröffentlichung |
|---|---|---|---|
| | | US 2009110710 A1 | 30-04-2009 |
| | | US 2011144767 A1 | 16-06-2011 |
| | | US 2012029653 A1 | 02-02-2012 |
| | | US 2012046758 A1 | 23-02-2012 |
| | | US 2012207808 A1 | 16-08-2012 |
| | | US 2014242044 A1 | 28-08-2014 |
| | | US 2014303744 A1 | 09-10-2014 |
| | | WO 2005004755 A1 | 20-01-2005 |
| EP 2774583 A1 | 10-09-2014 | CN 104027160 A | 10-09-2014 |
| | | EP 2774583 A1 | 10-09-2014 |
| | | US 2014257232 A1 | 11-09-2014 |

EPO FORM P0461

Für nähere Einzelheiten zu diesem Anhang : siehe Amtsblatt des Europäischen Patentamts, Nr.12/82

Seite 2 von 2

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 8034034 B2
- US 6582438 B
- WO 2013063706 A